# FASCICULE DE BREVET EUROPEEN

(11) **EP 0 726 760 B1**
(45) Date de publication et mention de la délivrance du brevet: **02.06.1999**
(21) Numéro de dépôt: 95900161.1
(22) Date de dépôt: 25.10.1994
(51) Int. Cl.: A61K 9/107, A61K 9/48, A61K 7/00

(54) **FORMULATION AUTO-EMULSIONNABLE FORMANT UNE HUILE DANS EAU**
SELBSTEMULGIERENDE FORMULIERUNG ZUR BILDUNG EINER ÖL-IN-WASSER-EMULSION
SELF-EMULSIBLE FORMULATION FORMING AN OIL-IN-WATER EMULSION

(30) Priorité: 02.11.1993 IL 10747193
(43) Date de publication de la demande: 21.08.1996
(73) Titulaire: YISSUM RESEARCH DEVELOPMENT COMPANY OF THE HEBREW UNIVERSITY OF JERUSALEM, F-92300 Levallois-Perret (FR)
(72) Inventeur: BENITA, Simon, 90805 Mevasseret Zion (IL); KLEINSTERN, Jackie, East Talpiot, 93807 Jerusalem (IL); GERSHANIK, Tatyana, Gilo, Jerusalem (IL)
(74) Mandataire: Desaix, Anne
(86) Numéro de dépôt international: FR9401239
(87) Numéro de publication internationale: WO9512384

(56) Documents cités:
- EP-A- 0 355 604
- EP-A- 0 394 847
- EP-A- 0 416 527
- WO-A-93/15736
- WO-A-93/18852
- FR-A- 2 372 635
- FR-A- 2 455 458

## Description

### Domaine de l'invention

La présente invention concerne une formulation auto-émulsionnable essentiellement hydrophobe, à savoir : une formulation qui, en mélange dans l'eau, se désintègre spontanément pour former une émulsion huile dans l'eau. La présente invention concerne aussi l'utilisation pharmaceutique d'une telle formulation à la fois (i) comme un véhicule de délivrance de médicaments lipophiles ou (ii) comme un précurseur pour la préparation d'une émulsion huile dans l'eau utile à son tour comme véhicule de délivrance de médicaments hydrophobes.

Une application de la formulation inventive est l'administration orale d'un médicament destiné à être absorbé dans le tube gastro-intestinal (GI) et, ensuite, atteindre l'organe cible par le sang ou le système lymphatique. Une telle forme d'administration sera dénommée ci-après "administration systémique par voie orale".

Une application particulière de la réalisation préférée précédente est l'administration systémique par voie orale d'un médicament tel que la physostigmine, le probucol, la cyclosporine A, la morphine base, la penclomédine et les autres.

La reconnaissance ci-après de l'art antérieur ne doit pas être interprétée comme l'admission que cet art est de quelque manière pertinent pour la brevetabilité de l'invention comme définie ici.

### Arrière-plan de l'invention

L'administration systémique par voie orale de médicaments, en général, est le mode préféré d'administration dans les régimes de traitement qui requièrent une administration répétée de médicaments pendant plusieurs périodes de temps. Tandis que l'administration systémique par voie orale est très efficace en ce qui concerne les médicaments solubles dans l'eau, il est prouvé qu'elle est une voie d'administration problématique pour les médicaments hydrophobes ou les médicaments ayant une solubilité aqueuse limitée comme la physostigmine base, l'isradipine, la virginiamycine, la cyclosporine A, la morphine, la buprénorphine, la nalorphine, le méthorfan, le probucol et autres.

Le faible effet systémique atteint avec les médicaments hydrophobes administrés oralement, résulte d'un certain nombre de facteurs. Premièrement, les médicaments hydrophobes ne sont pas solubles dans l'eau et forment une phase séparée dans les solutions aqueuses et ainsi ne sont pas facilement disponibles pour l'absorption par les parois du tube gastro-intestinal.

De plus, certains médicaments hydrophobes qui sont absorbés primairement par les parois de l'intestin grêle, particulièrement à travers le jéjunum, peuvent subir un effet nommé "effet de premier passage", c'est-à-dire un passage de médicaments dans le foie préalablement à atteindre le système sanguin.

L'effet total de ces facteurs est que seulement une faible, souvent non efficace, quantité de médicaments hydrophobes administrés atteint finalement l'(es) organe(s) cible, c'est-à-dire que les médicaments hydrophobes administrés oralement ont généralement une faible biodisponibilité.

Ceci peut être surmonté en augmentant la dose de médicament, mais une telle augmentation peut, cependant, entraîner une incidence accrue des effets secondaires, étant donné l'absorption erratique et variable inter-individus.

Il y a eu des propositions variées pour augmenter la biodisponibilité des médicaments hydrophobes. Par exemple, des études précédentes utilisant un acide oléique contenant un médicament lipophile dissout, ont démontré un effet bénéfique sur la biodisponibilité du médicament (Stella et all., 1978. J.Pharm.Sci, 67, 1375-1377). Récemment, on a montré que la biodisponibilité du propranolol, après administration orale, peut être amélioré' en dissolvant le médicament dans une formulation lipidique contenant principalement de l'acide oléique et en le conditionnant dans une capsule en gélatine dure gastro-protégée (Barnwell et al, 1992, Int. J. Pharmaceutics, 88, 423-432).

Des émulsions ont été proposées comme vecteurs dans les formulations orales de médicaments hydrophobes en général (Pal et al, 1984, J. Int. Pharm, 33, 99-104; Myers et al, 1992, Int. J. Pharm, 78, 217-226) et pour des médicaments comme la physostigmine en particulier (Rubinstein et al, 1991, J. Pharm. Res, 80, 643-647; Friedman et al, 1989, Drug Design and Delivery, 4, 135-142; Benita et al, 1989, Drug Delivery Design 4, 143-153). Les particules colloïdales de l'émulsion qui transporte le médicament sont absorbées dans le jéjunum et sont probablement évacuées principalement par la lymphe à travers le canal thoracique, passant par dérivation ainsi le foie et réduisant grandement "l'effet de premier passage". En effet, la biodisponibilité orale de plusieurs médicaments lipophiles a été montrée comme étant quelque peu améliorée en utilisant des émulsions comme véhicules pour leur administration systémique par voie orale.

Cependant, dans de nombreux cas, les formulations émulsionables n'ont pas apporté d'amélioration dans la biodisponibilité de médicaments hydrophobes face à leur administration dans des solutions aqueuse.

Ceci est particulièrement le cas au regard des médicaments comme la physostigmine, qui est un médicament amphiphile dans l'émulsion dans le film interfacial huile/eau.

Pendant le passage d'une émulsion contenant de la physostigmine à travers le tube digestif, l'émulsion, qui est pratiquement infiniment diluée, libère rapidement le médicament contenu dans celle-ci. Ce problème est considérablement augmenté par la très forte acidité dans l'estomac, qui a tendance à réduire la stabilité des particules colloïdales de l'émulsion.

Certaines solutions lipidiques, en raison de leurs ingrédients, ont la capacité de subir une émulsification spontanée quand elles sont introduites dans une phase aqueuse après une agitation douce fournissant des émulsions huile dans l'eau. De telles solutions lipidiques sont définies dans la littérature comme systèmes de délivrance auto-émulsionnables (Charman et al, 1992, Pharm. Res, 9, 87-93). Des systèmes de délivrance auto-émulsionnables ont été formulés en employant des huiles de triglycérides à chaîne moyenne et des agents tensioactifs non-ioniques qui, selon leur nature exacte, peuvent former la base d'un système de délivrance de médicament auto-émulsionnable (Pouton, 1985a, Int. J. Pharm 27, 335-348; Pouton 1985b, Int. J. Pharm, 37, 1P; Pouton et al, 1987, Proc. Int. Symp. Control. Rel, Bioacta. Mater, 14, 113-117; Wakerly et al, 1986, ACS. Symp. Ser. 311, 242-255; Wakerly et al, 1987, J. Pharm. Sci, 67, 1375-1377). Ces formulations peuvent être encapsulées dans des capsules molles de gélatine ou dans des capsules de gélatine dures scellées pour fournir des systèmes de dosage unitaire précis et commodes.

Des études antérieures dans l'intestin grêle ont établi clairement que l'intérieur des cellules d'absorption est négatif par rapport à la solution muqueuse (Csaky, IL (Ed), Handbook of Experimental Pharmacology, Vol 70, Springer-Verloy, Berlin, (1984), 324-325). On a également décrit le fait que certains médicaments cationiques hydrophobes complètement ionisés sur toute l'étendue de pH du tube digestif, sont absorbés rapidement en dépit de leur faible solubilité dans l'eau (Iseky, K. Hirano,T. Fukushi,Y. KITAMURA,Y. Miyazaki,S. Takada,K. Sugawara,M. Saiton,H and Miyazaki, K (1992), J.Pharm.Pharmacol. 44:9, 724-726: Saiton, H. Kawai, S. Iseki, K. Myazaki, K and Arita, T. (1988), J.Pharm.Pharmacol, 41, 200-202). En outre, certains composés endogènes se lient aux surfaces endothéliales par leur groupe NH₂ termina indiquant l'importance physiologique des interactions électrostatiques.

La formation de gouttelettes huileuses contenant un médicament dissout amène la distribution du médicament à travers tout le tube digestif tout en fournissant une large surface interfaciale pour la répartition du médicament entre l'huile et la phase aqueuse environnante. Ainsi, pour des médicaments ayant une solubilité aqueuse limitée qui sont peu absorbés dans le tube digestif, la désintégration spontanée de la phase lipidique en gouttelettes huileuses très finement dispersées, peut offrir une amélioration à la fois dans la vitesse et la proportion de l'absorption. Tous les systèmes de délivrance de médicament auto-émulsionnants connus actuellement contiennent des concentrations importantes de tensioactifs (jusqu'à 50%) qui sont soit non-ioniques (comme le Tween^{(R)} ou le Span^{(R)}) ou des tensioactifs anioniques (comme les phospholipides), ce qui entraîne la formation de gouttelettes huileuses ayant une charge soit neutre, soit électronégative.

Dans une publication récente (Elbaz et al, 1993, Int. J. Pharm, 96, R1-R6), un système de délivrance de médicament en émulsion a été décrit dans lequel les particules colloïdales portent une charge positive. Cependant, aucune menton n'a été faite, dans cette publication, de la possibilité d'employer de telles émulsions comme véhicule de délivrance de médicament systémique par voie orale.

Le document FR-A-2 455 458 décrit une composition se présentant sous la forme de liposomes et contenant une substance active comme l'insuline. La composition comprend stéarylamine, huile de graines de coton et lécithine; elle a une bonne résistance mécanique et elle présente une libération contrôlée.

Le document EP-A-0 394 847 décrit une composition herbicide auto-émulsionnable et facile à dissoudre. Ladite composition comprend une substance active (herbicide), une oleylamine, une huile, des esters d'acides gras avec un alcanol et agent tensioactif.

Le document EP-A-0 416 527 décrit une composition se présentant sous la forme de liposomes de charge positive et contenant une substance active pour administration orale. La composition comprend une phosphatidile choline, une oleylamine, et une prostaglandine. Ladite composition présente une libération contrôlée et elle est encapsulée dans une capsule gastro-protégée.

Le document WO-A-9 318 852 décrit une émulsion huile dans l'eau pharmaceutique pour administration orale contenant des particules de charge positive. La composition comprend une huile qui est enfermée dans un film comprenant un tensioactif non-ionique, une stéarylamine ou une aoleylamine et un principe actif.

C'est un objet de la présente invention de fournir une formulation auto-émulsionnable utile comme système de délivrance de médicament pour les médicaments lipophiles.

C'est un objet supplémentaire de l'invention de fournir un nouveau système de délivrance de médicament pour l'administration orale systémique de médicaments lipophiles.

C'est encore un autre objet de l'invention de fournir un procédé de production d'une émulsion par l'emploi d'une préparation huileuse auto-émulsionnante.

### Description générale de l'invention

Conformément à l'invention, une nouvelle formulation huileuse auto-émulsionnable (SEOF) est fournie. Lorsque la formulation selon l'invention est mélangée avec une solution aqueuse et que le mélange est agité, une émulsion huile dans l'eau se forme. Conformément à une réalisation préférée de l'invention, les gouttelettes dans l'émulsion ainsi formées ont un diamètre en-dessous d'environ 0,2µm.

Les émulsions ayant des gouttelettes minuscules comme celles que l'on peut obtenir conformément à l'invention, ont été jusqu'ici obtenues seulement en employant une procédure d'homogénéisation complexe impliquant l'emploi d'un équipement compliqué (voir par exemple, Benita et al, 1989, Drug Delivery Designn 4, 143-153).

Une caractéristique supplémentaire des SEOF de l'invention est le fait que les gouttelettes dans l'émulsion formée sont chargées positivement au contraire des émulsions chargées négativement, réalisées avec les SEOF de l'art antérieur.

Les SEOF de l'invention comprennent un support huileux qui peut être une huile de triglycéride à chaîne moyenne (MCT), un triglycéride à longue chaîne (LCT) et un dérivé d'acide gras huileux. En supplément, les SEOF comprennent aussi un lipide cationique. On a trouvé, conformément à l'invention, que l'addition d'un alcool lipophile est nécessaire pour obtenir des gouttelettes en émulsion dans la gamme inférieure au micron (<1µm). L'incorporation d'un alcool lipophile dans les SEOF de l'invention est ainsi préférée.

La présente invention ainsi procure une formulation huileuse auto-émulsionnable (SEOF) contenant un composant huileux et un agent tensioactif, le SEOF étant caractérisé en ce que le composant huileux contient un vecteur huileux et un lipide cationique et, éventuellement, un alcool lipophile, l'émulsion huile dans l'eau qui se forme par mélange du SEOF ayant des gouttelettes huileuses qui sont chargées positivement.

Les SEOF de l'invention peuvent être employés comme véhicule de délivrance pour des médicaments hydrophobes. Lorsqu'un tel véhicule de délivrance ayant les médicaments dissous dedans, vient en contact avec un liquide corporel, il s'émulsionne spontanément en formant des gouttelettes huileuses minuscules avec le médicament contenu à l'intérieur.

Un exemple spécifique d'un tel véhicule de délivrance est celui qui est destiné à l'administration systémique par voie orale de médicaments hydrophobes. Pour une telle administration, le SEOF ayant les médicaments dissous, est de préférence encapsulé.

La présente invention ainsi procure une composition pharmaceutique contenant une quantité efficace d'un médicament hydrophobe dissout dans un vecteur liquide, la préparation étant caractérisée en ce que ledit vecteur est le SEOF décrit ci-dessus.

Des réalisations spécifiques de ladite préparation sont les préparations systémiques pour la voie orale.

Le terme "quantité efficace" doit être compris comme signifiant une dose du médicament efficace pour exercer un effet thérapeutique. Pour une préparation orale de l'invention, le terme "quantité efficace" signifie une dose de médicament qui, après absorption dans le corps à travers les parois du tube digestif, procure une concentration de médicament dans le sang qui est efficace pour exercer un effet thérapeutique sur un organe cible.

L'invention fournit aussi une méthode d'administration d'un médicament hydrophobe aux emplacements dans le corps, par exemple le tube digestif, dans le sang où la préparation entre en contact avec les liquides corporels, comprenant l'administration du médicament dans un véhicule huileux qui est le SEOF décrit ci-dessus.

Un autre emploi du SEOF de l'invention est dans un procédé de production d'émulsions, en particulier celles ayant des gouttelettes dans la gamme inférieure au micromètre (émulsions submicroniques). Une réalisation spécifique est dans la préparation d'émulsions contenant un médicament hydrophobe et destinée à être utilisée comme préparations pharmaceutiques. Ainsi, la présente invention procure un procédé pour la production d'une émulsion qui comprend le mélange dudit SEOF avec une solution aqueuse et le fait d'ajouter le mélange.

Lorsque l'émulsion doit être utilisée comme un vecteur de médicaments hydrophobes, la formulation auto-émulsionnable (SEOF) doit avoir ledit médicament dissout dans celle-ci.

### Description detaillée de l'invention

Conformément à l'invention, une nouvelle formulation huileuse auto-émulsionnable a été préparée. Cette formulation SEOF peut être utilisée dans des préparations pharmaceutiques comme un vecteur de médicaments hydrophobes ou peut être employée comme précurseur pour la préparation d'émulsions, en particulier d'émulsion inférieure à un micromètre.

Les formulations SEOF de l'invention comprennent un agent tensioactif et un composant huileux contenant un lipide cationique et au moins un MCT, un LCT et un dérivé d'acide gras huileux. Le composant huileux contient un alcool gras huileux comme ingrédient éventuel. En outre, le composant huileux peut aussi inclure d'autres ingrédients comme cela sera spécifié ci-dessous. Evidemment, tous les principes actifs utilisés doivent être physiologiquement compatible.

L'huile MCT est une huile triglycéridique dans laquelle la chaîne hydrocarbonée a en moyenne environ 8 à 12 atomes de carbone. Des exemples d'huiles MCT sont le TCM ^{(TM)} (Société des Oléagineux France) qui est un mélange de triglycérides dans lesquels environ 95% des chaînes d'acide gras ont, soit 8 ou 10 atomes de carbone, ou bien le Myglyol 812 ^{(TM)} (Dynamit Nobel Suède) qui est un mélange de triesters de glycerol et d'acides caprylique et caprique.

L'huile LCT est une huile triglycéridique dans laquelle la chaîne hydrocarbonée a une longueur de chaîne moyenne au-dessus de 12 atomes de carbone. Des exemples d'huiles LCT qui peuvent être employées conformément à la présente invention sont l'huile d'arachide, l'huile de carthame, l'huile de sésame, l'huile de soja, l'huile de graines de coton, l'huile d'olive.

Les dérivés d'acides gras huileux peuvent être divers acides gras substitués lipophiles, par exemple, des esters avec des alcanols, les exemples étant les esters méthyliques ou éthyliques d'acide gras comme l'oléate d'éthyle.

Les lipides cationiques sont des lipides qui ont un groupe polaire chargé positivement. Des exemples de lipides cationiques sont les amines à chaîne alcoyle grasses en C₁₀ à C₂₄ et les alcanoylamines grasses en C₁₂ à C₂₄, les alcoylamines grasses en C₁₂ à C₁₈ et les alcanoylamines grasses en C₁₂ à C₁₈ étant préférées. Des exemples spécifiques de lipides cationiques sont la stéarylamine et l'oleylamine. En supplément aux lipides cationiques, le composant huileux peut aussi inclure des agents tensioactifs cationiques tels qu'un ester cationique de cholestérol et des dérivés cationiques de cholestérol, par exemple, le betainate de cholestérol.

Les alcools gras huileux comprennent divers alcools lipophiliques comme des alcools d'acides gras, par exemple, l'alcool oleylique ou des alcools aryliques, par exemple, l'alcool benzylique, l'alcool éthylique ou tout autre alcool non toxique lipophile.

En supplément aux ingrédients désignés ci-dessus, ledit composant huileux peut aussi comprendre divers autres ingrédients huileux y compris d'autres types d'huile végétale, d'huile minérale ou de myristate d'isopropyle. En outre, le composant huileux peut aussi inclure des lipides neutres ou anioniques, la quantité de lipides anioniques étant telle de façon à ne pas éliminer complètement la charge positive des gouttelettes d'émulsion.

Les agents tensioactifs employés dans la formulation SEOF peuvent être tout agent parmi ceux connus "per se". L'agent tensioactif est de préférence un agent tensioactif non ionique et, peut être, par exemple, un Tween, par exemple le Tween 80 et le Tween 85; des Span, par exemple Span 80 ou un glycéride polyoxyéthylénique glycosylé, par exemple Labrafil M1944 CS ^{(TM)} (Gattefosse Corp, USA).

D'autres exemples d'agents tensioactifs non-ioniques sont les esters de sorbitol et d'acide gras, comme le monooléate de sorbitanne et les sucrose-esters huileux comme le mono-, di- ou tripalmitate de sucrose. En supplément aux agents tensioactifs non-ioniques, on peut utiliser aussi des agents tensioactifs ioniques comme les phospholipides, la quantité de tels agents tensioactifs ioniques doit être telle qu'elle n'élimine pas la charge positive des gouttelettes d'émulsion. De temps en temps, il est utile d'employer une combinaison de différents agents tensioactifs comme une combinaison de Tween 80 et de Span 80, ou bien de Tween 85 et de Span 80.

L'application pharmaceutique typique de la formulation SEOF de l'invention est l'administration orale systémique de médicaments hydrophobes. Pour cette administration, la formulation SEOF ayant le médicament dissout à l'intérieur, va être de préférence encapsulé dans une capsule molle ou dure de gélatine scellée. La capsule est typiquement d'un genre qui est dissout dans une région particulière du tractus gastro-intestinal en libérant à son contenu.

Un exemple d'une telle capsule est une capsule molle ou dure de gélatine gastro-protégée. L'enrobage entérique comme c'est connu "per se", est un enrobage avec une substance ou une combinaison de substances qui résiste à la dissolution dans le suc gastrique mais qui se désintègre dans les intestins. Des exemples d'enrobages entériques sont le phtalate d'hydroxypropyl méthylcellulose BP. Par suite de l'enrobage entérique, la capsule est résistante à la dissolution dans les parties supérieures du tube digestif et ainsi, ne se dissout que dans l'intestin, par exemple, par suite du contact avec les acides biliaires et les sels dans le jéjunum. Les gouttelettes d'émulsion, particulièrement celles contenant du LCT, par exemple de l'huile de soja, sont vraisemblablement absorbées dans le jéjunum par l'intermédiaire du système lymphatique dans les chylomicrons qui sont écartés de l'intestin grêle à travers le canal thoracique, puis effectuant un by-pass du foie. Une telle voie d'absorption réduit ainsi d'une manière importante l'effet de premier passage de la dégradation du médicament dans le foie.

Les capsules commercialement accessibles, s'échelonnent en taille jusqu'à celles ayant un volume interne d'environ 0,7 ml. A partir des expériences conduites conformément à la présente invention, il va devenir évident que ce volume est suffisant pour la délivrance d'une quantité efficace de médicament, comme la physostigmine, de façon à réaliser un effet systémique.

Pour l'emploi pharmaceutique de la formulation SEOF de l'invention, des agents conservateurs comme le méthylparaben, le propylparaben, le butylparaben ou une combinaison de ceux-ci ainsi que des agents anti-oxydants comme le gallate de propyle, le BHT ou le dimercaptol peuvent être employés. En outre, préalablement à l'emploi, la préparation est de préférence stérilisée par filtration.

L'invention va maintenant être davantage illustrée dans les exemples suivants et les dessins annexes.

### Brève description des dessins

Dans les dessins :
Figure 1 : montre la taille moyenne d'une gouttelette en fonction du faux de dilution de la formulation SEOF dans la phase aqueuse. La formulation SEOF était formée de 25% de Tween 80, 12,5% d'alcool benzylique, 2,5% d'oleylamine et de l'oléate d'éthyle pour 100%. Les détails de ces expériences sont décrits dans l'exemple IV.
Figure 2 : montre les résultats d'une expérience similaire à celle montrée à la figure 1, également décrite dans l'exemple IV, avec la différence étant que la concentration de Tween 80 était de 35%.
Figure 3: montre les résultats d'une expérience décrite à l'exemple IV dans laquelle la taille moyenne de gouttelettes a été testée par rapport à la durée dans trois dilutions différentes de la formulation SEOF en phase aqueuse. La formulation SEOF était formée de 25% de Tween 80, 12% d'alcool benzylique, 2,5% d'oleylamine et d'oléate d'éthyle pour 100%. Le niveau de pH de la phase aqueuse était de 5,0.
Figure 4 : montre les résultats d'une expérience similaire à celle de la figure 3, également décrite à l'exemple IV avec la différence étant en ce que le niveau du pH de la phase aqueuse était de 2,5 (ajusté avec un tampon phtalate).
Figure 5 : montre les résultats d'une expérience dans laquelle l'inhibition de la choline esterase en fonction du temps suivant l'administration PO de trois formulations différentes de physostigmine.
Figure 6 : montre les concentrations sériques de Progestérone après administration de SEOF positif et négatif contenant le médicament ainsi que l'expérience avec la progestérone en suspension dans l'eau et une formulation à blanc.

### Méthodes expérimentales

### 1. Evaluation de la taille des particules

La taille des gouttelettes de particule de la formulation SEOF, se désintégrant a été déterminée en utilisant un appareil super-Nanosizer (Coulter N4) dans lequel les tailles de particules sont évaluées par spectroscopie de corrélation par photons. Préalablement à I'évaluation, les préparations ont été diluées en employant une solution de glycérol à 2,25% filtrée et traitée aux ultrasons.

### 2. Charge de l'émulsion

La charge de l'émulsion a été déterminée par mobilité électrophorétique. Une émulsion de grasse standard chargée négativement (Intralipid^{(R)} Kabi-Vitrum, Suède) immergée dans la solution de glycérol à 2,25% se déplace dans la direction de l'électrode positive étant donné la charge négative des gouttelettes huileuses en dispersion. Les diverses formulations huileuses émulsionnées (dans le suc gastrique et dans le suc intestinal USP) migraient dans la direction opposée en direction de l'électrode négative validant la charge positive des gouttelettes d'huile.

### 3. Potentiel Zêta

Le potentiel Zêta a été mesuré en employant la technique d'électrophorèse limite en déplacement. La valeur du potentiel Zêta des gouttelettes d'huile formées spontanément, a été déterminée en utilisant un Zetasizer^{(TM)} (Delsa 440 Coulter) en employant une solution de glycérol à 2,25°/°° comme diluant.

### 4. Observations visuelles

Le degré de stabilité de la formulation et le degré de séparation de phase dans l'émulsion dérivée de ces formulations ont été déterminés visuellement à des intervalles de temps donnés. Aucun changement visible n'a été enregistré.

### EXEMPLE I

### Préparation de la formulation SEOF

Les SEOF ont été préparés avec les étapes suivantes :
**1.** l'alcool gras et la plus grande partie du vecteur huileux ont été mélangés dans une fiole.
**2.** un agent tensioactif a été ensuite ajouté et rendu soluble par agitation
**3.** le lipide cationique a alors été ajouté et dissout dans le mélange ci-dessus. Dans le cas de la stéarylamine, un léger chauffage vers environ 37°C a été nécessaire pour solubiliser ce composant.
**4.** le poids final de la composition a été complété par addition du vecteur huileux restant.
**5.** le médicament peut être dissout soit dans l'alcool gras préalablement à son addition au mélange, soit ajouté après l'incorporation des autres ingrédients de la formulation avant de compléter au poids final avec le vecteur huileux.
**6.** seulement dans les exemples VI-X, le médicament y était inclus. Les exemples II-V ne contenaient pas de médicament.

Pour la formation de l'émulsion, la formulation préparée de SEOF a été mélangée avec de l'eau deux fois distillée ou avec du jus gastrique et intestinal artificiel (USP).

### EXEMPLE II

### Les SEOF qui se désintègrent en gouttelettes huileuses ayant un diamètre inférieur à 250 µm

Une gamme de SEOF a été préparée à partir des ingrédients suivants : Span, Tween 85, stéarylamine et huile d'arachide. Le tableau suivant donne la liste des quatre ingrédients montrant la gamme de concentration testée et la gamme de concentration préférée et une concentration typique pour chaque ingrédient qui produit une taille de particule minimale.

**Tableau I**

| Constituants | Concentrations préférées (%, w/w) | Gamme de concentrations (%, w/w) | Concentrations typiques (%, w/w) |
|---|---|---|---|
| Span 80 | 25 - 30 | 15 - 40 | 25 |
| Tween 85 | 4 - 5 | 1 - 10 | 3 |
| Stearylamine | 2.5 - 3 | 1 - 3 | 3 |
| Huile d'arachide | | | to 100 |

Chacune des formulations était transparente sous forme liquide à 37°C et devenait opalescente et visqueuse à température ambiante. Les émulsions qui se sont formées à partir de ces formulations sont demeurées stables à température ambiante après quelques heures. La taille des particules des gouttelettes huileuses dans les émulsions, après agitation douce, s'est échelonnée de 50 à 250 µm.

En supplément à l'huile d'arachide, d'autres huiles LCT comprenant l'huile de carthame, l'huile de sésame, l'huile de soja, l'huile de graines de coton et l'huile d'olive ont été testées.

Les émulsions à l'intérieur de la gamme ci-dessus (50-250µm) ont été réalisées avec toutes ces huiles LCT. Les émulsions optimales ayant des gouttelettes à la partie inférieure de la gamme citée ci-dessus ont été obtenues avec les huiles d'arachide et de carthame.

En outre, l'huile MCT aussi a été testée à la place de l'huile d'arachide et on a montré qu'elle fournissait aussi des gouttelettes d'une taille à l'intérieur de la même gamme.

### EXEMPLE III

### Les SEOF qui se désintègrent en gouttelettes d'huile submicroniques

Les SEOF ont été préparés à partir des constituants suivants : Tween 80, Span 80, alcool oleylique, oleylamine et oléate d'éthyle. Les constituants d'une formulation typique, la gamme de concentration testée, pour chaque constituant, la gamme de concentration préférée et une concentration typique fournissant des gouttelettes de taille minimale, sont mis en évidence dans le Tableau II

**Tableau II**

| Constituants | Concentrations préférées (%, w/w) | Gamme de concentrations (%, w/w) | Concentrations typiques (%, w/w) |
|---|---|---|---|
| Tween 80 | 25 - 30 | 15 - 40 | 25 |
| Span 80 | 1.5 - 2 | 0.5 - 3 | 1.5 |
| Alcool oleylique | 5 - 8 | 0 - 10 | 7.5 |
| Oleylamine | 2 - 2.5 | 1 - 3 | 2.5 |
| Oléate d'éthyle | | | to 100 |

La taille des gouttelettes des émulsions qui étaient formées à partir de ces SEOF s'échelonnait de 160 à 200 nm de diamètre avec une déviation standard de 60-70 nm. Les gouttelettes huileuses étaient chargées positivement.

Les émulsions étaient toutes stables à température ambiante pendant plusieurs semaines.

On a trouvé que la concentration en alcool oleylique dans la solution lipidique affectait la taille finale des gouttelettes. Les résultats sont montrés dans le tableau III suivant :

**Tableau III**

| Concentration en alcool oléylique (% w/w) | Diamètre des gouttelettes (nm) |
|---|---|
| 5 | 160 ± 60 |
| 10 | 200 ± 63 |
| 12.5 | 650 ± 900 |

Quand on utilisait du Tween 85 au lieu du Tween 80, une augmentation de la concentration en oleylamine jusqu'à 7-8% était nécessaire pour réaliser une émulsion positive. La taille de particules finale des gouttelettes dispersées résultant dans ce cas s'échelonnait de 1 à 2 µm.

L'Oléate d'éthyle était le seul solvant de ceux testés qui était capable de produire des émulsions dans la gamme submicronique.

### EXEMPLE IV

### Les SEOF qui se désintègrent en gouttelettes huileuses s'échelonnant de 20 à 200 nm.

Les SEOF étaient préparés à partir des ingrédients suivants : Tween 80, Alcool benzylique, oleylamine et oléate d'éthyle.

Les ingrédients dans l'échelle de concentration testée de chacun sont montrés dans le tableau IV.

**Tableau IV**

| Constituants | Gamme de concentration (%, w/w) |
|---|---|
| Tween 80 | 25 - 40 |
| Alcool benzylique | 0.5 - 50 |
| Oleylamine | 2.5 |
| Oléate d'éthyle | pour 100 |

Une formulation de ceux-ci qui contenait moins de 0,5% d'alcool benzylique n'était pas stable. Quand plus de 50% d'alcool benzylique était incorporé, aucune émulsion submicronique stable n'était réalisée.

Le tableau V suivant montre la taille de particules comme une fonction de la concentration en Tween 80 et en alcool benzylique (sous dilution constante avec la phase aqueuse de 1:1)

**Tableau V**

| | | | | | | |
|---|---|---|---|---|---|---|
| Tween 80 (%, w/w) | 25 | 25 | 25 | 25 | 35 | 40 |
| Alcool benzylique (%, w/w) | 5 - 12 | 25 | 37.5 | 50 | 45 | 40 |
| Taille des particules (nm) | 191±60 | 175±63 | 110±38 | 86±30 | 37±14 | < 12 |

Comme on peut le voir, le plus petit diamètre de gouttelette est atteint avec une formulation contenant 40% de Tween 80 et une formulation contenant 40% d'alcool benzylique

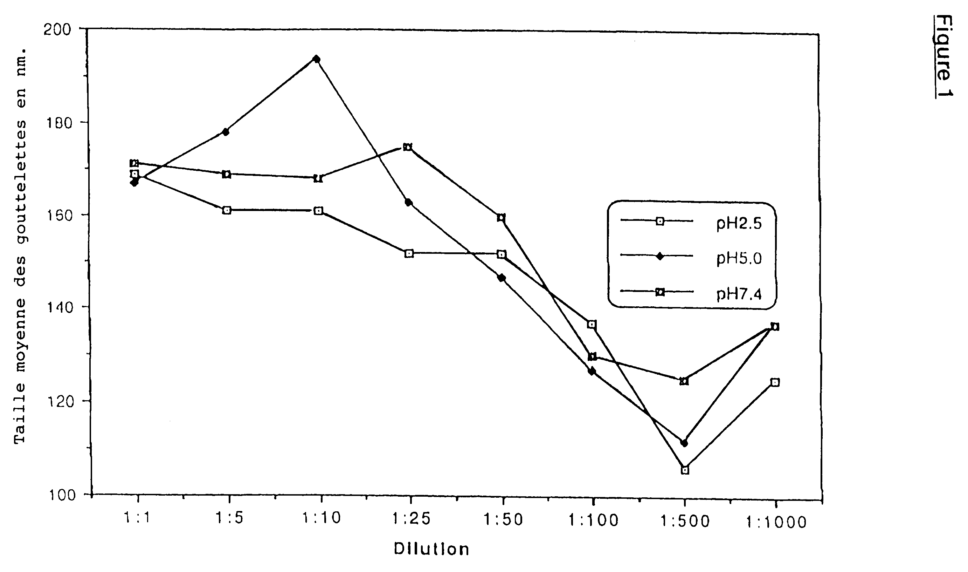

La Figure 1 montre les résultats obtenus avec un SEOF qui est formé de 25% de Tween 80,12,5% d'alcool benzylique, 2,5% d'oleylamine et d'oléate d'éthyle ajoutés pour un total de 100%. Comme le montre la figure 1, la taille des particules a diminué avec une dilution de phase aqueuse augmentée jusqu'à une dilution d'environ 1:500. Une dilution supplémentaire à 1:1000 a amené à une légère augmentation de la taille des gouttelettes.

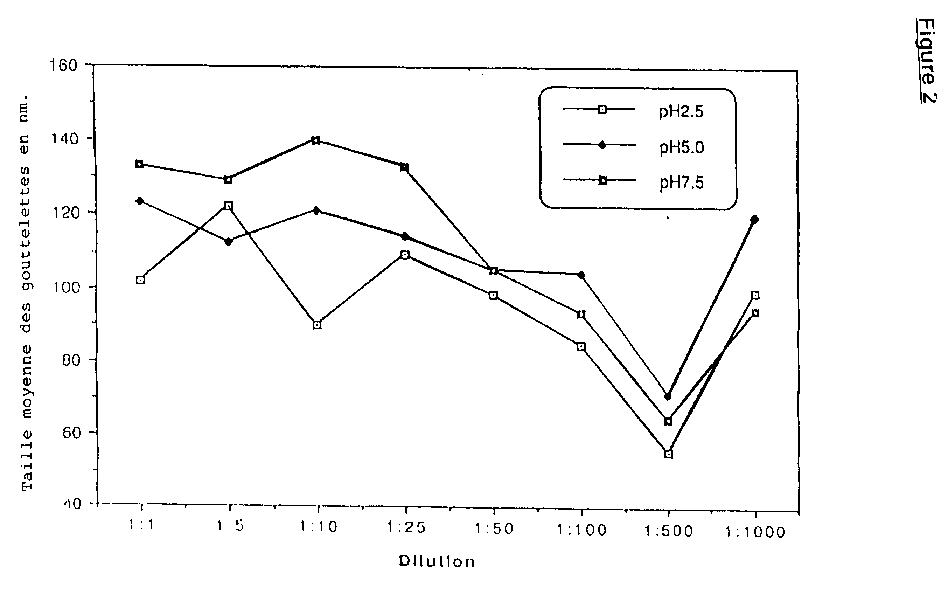

Comme le montre la Figure 2, le même type de comportement était aussi constaté avec un SEOF différent contenant 35% de Tween 80 au lieu de 25% comme dans le SEOF de la figure 1.

La Figure 1, la Figure 2 et le tableau VI démontrent qu'une auto-émulsification efficace ne montre pas une dépendance élevée du pH, mais une certaine tendance à l'élargissement de la taille des gouttelettes était observée comme une fonction d'augmentation de pH.

Influence du pH sur la taille moyenne des gouttelettes comme une fonction du pH et de la durée.

**Tableau VI**

| pH | dilution | 0 jour | 1 jour | 7 jours | 34 jours |
|---|---|---|---|---|---|
| 2.5 | 1/500 | 105±36 | 113±25 | 120±33 | 137±45 |
| | 1/1000 | 125±45 | 127±37 | 134±42 | 157±52 |
| 5.0 | 1/500 | 112±34 | 120±36 | 125±36 | 136±43 |
| | 1/1000 | 130±46 | 139±46 | 150±44 | 157±52 |
| 7.4 | 1/500 | 127±44 | 137±44 | 145±44 | 198±large |
| | 1/1000 | 137±48 | 146±52 | 151±56 | 188±large |

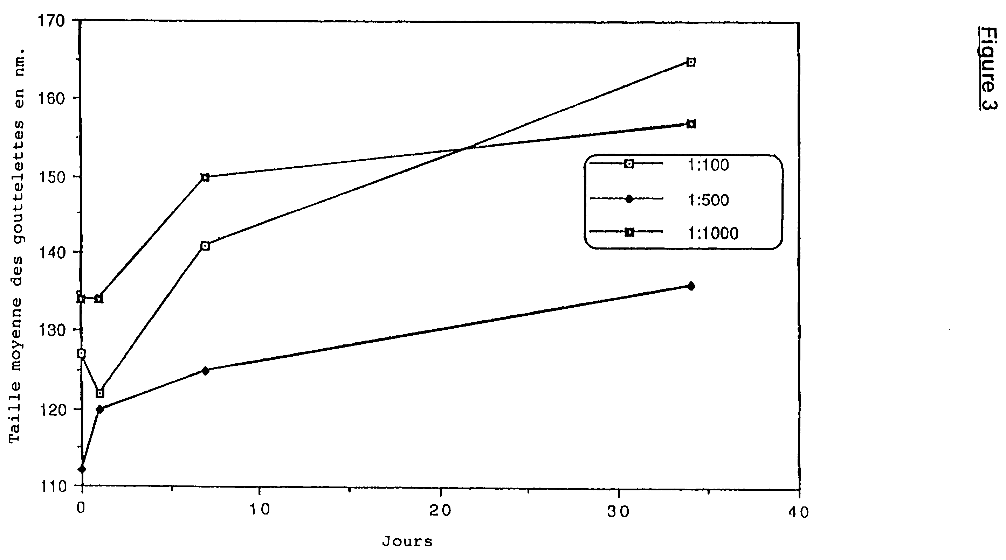

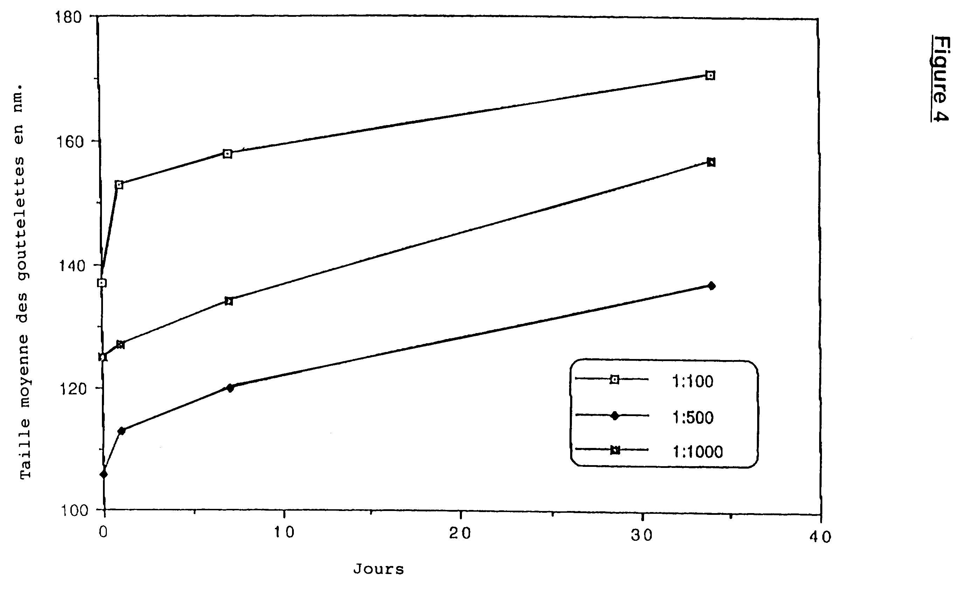

Référence est faite aux figures 3 et 4 montrant les résultats d'une expérience dans laquelle la stabilité des émulsions, comme mise en évidence par la taille moyenne de gouttelettes, a été testée au fur et à mesure des temps. Les SEOF, dans les deux cas, étaient formés de 25% de Tween 80, 12,5% d'alcool benzylique (BA), 2,5% d'oleylamine (OA) et d'oléate d'éthyle (EO) ajoutés pour faire 100%.

Dans l'expérience montrée à la figure 3, le pH était 5.0 tandis que le pH dans l'expérience de la figure 4 était 2.5. Comme il est montré, les émulsions qui s'étaient formées étaient stables pendant plus de 30 jours dans les conditions testées. Il doit être noté que sans une dilution de moins de 1:1000, les émulsions étaient stables pendant seulement une semaine environ. De plus, à un pH de la phase aqueuse de plus de 7.4, les émulsions étaient également stables pendant une période d'une semaine seulement.

### EXEMPLE V

### Le potentiel Zêta des gouttelettes, produit par SEOF

Les mesures quantitatives du potentiel Zêta ont été composées avec des émulsions, préparées à partir de SEOF, qui étaient décrites à l'exemple III. Les émulsions comprenaient différentes quantités d'oleylamine (0.5%). Le tableau VII montre les potentiels Zêta de ces émulsions, les émulsions résultant de la dilution des SEOF (1:10) comprenant différentes quantités d'oleylamine (0-0,5%), mesurées dans une solution de glycérine à 2,25%.

Comme il est montré, les SEOF, qui incluaient de l'oleylamine étaient capables de produire des gouttelettes portant une charge positive.

**Tableau VII**

| **Mesures au potentiel Zêta des émulsions produites par dilution de SEDDS avec une solution de glycérine à 2,25%** | | |
|---|---|---|
| | Formulation (%, w/w) | Valeur du potentiel zêta à 25°C (mV) |
| Tween 80 | 25 | - 8.4 |
| BA | 12.5 | |
| EO | pour 100 | |
| Tween 80 | 25 | + 15.8 |
| BA | 12.5 | |
| OA | 2.5 | |
| EO | pour 100 | |
| Tween 80 | 25 | + 33.3 |
| BA | 12.5 | |
| OA | 5 | |
| EO | pour 100 | |

### EXEMPLE VI

### Les SEOF avec les médicaments

La physostigmine, la cyclosporine A, le probucol et l'insuline (l'insuline était finement dispersée dans la formulation huileuse par exposition aux ultrasons pendant 5 mn en utilisant un bain d'ultrasons) étaient incorporées dans les SEOF et les formulations sont montrées au tableau VIII suivant :

**Tableau VIII**

| | Constituants | Concentration (%, w/w) |
|---|---|---|
| **1** | Physostigmine Salicylate 0.6% | 25 % |
| | Span 80 | 25 % |
| | Tween 85 | 5 % |
| | Stearylamine | 3 % |
| | Huile d'arachide | qsp 100 % |
| **2** | Cyclosporine A | 5 % |
| | Tween 80 | 25 % |
| | Span 80 | 1.5 % |
| | Alcool oleylique | 7.5 % |
| | Oleylamine | 2.5 % |
| | Oléate d'éthyle | qsp 100 % |
| **3** | Probucol | 4 % |
| | Tween 80 | 25 % |
| | Alcool benzylique | 50 % |
| | Oleylamine | 2.5 % |
| | Oléate d'éthyle | qsp 100 % |
| **4** | Insuline | 0.5 % |
| | Tween 80 | 25 % |
| | Alcool benzylique | 12.5 % |
| | Oleylamine | 2.5 % |
| | Oléate d'éthyle | qsp 100 % |
| **5** | Progesterone | 10 % |
| | Tween 80 | 25 % |
| | Alcool benzylique | 30 % |
| | Oleylamine | 2.5 % |
| | Oléate d'éthyle | qsp 100 % |

### EXEMPLE VII

### Inhibition de la choline esterase par administration orale de physostigmine à des rats.

L'effet pharmacologique de la physostigmine (PS) administrée oralement sur des rats a été testée en modifiant une méthode précédemment décrite par Steffens A.B (969, Physiology and Behavior 4, 833-836). Des mâles rats Sabra ont été cannulés d'une manière chronique par la veine jugulaire un jour avant l'expérience, permettant un échantillonnage fréquent de sang d'un rat non anesthésié. Chaque groupe de rats éveillés (3-4 animaux dans chaque groupe) a reçu per os (PO) une ou trois formulations de physostigmine (PS). Toutes les formulations ont été administrées à une dose de PS de 0.5 mg/kg. 0.1 ml d'échantillons de sang ont été prélevés à des intervalles de temps spécifiques jusqu'à 5 heures après l'administration du médicament et analysés pour l'activité choline estérasique selon l'essai radiométrique de Johnson & Russel (Johnson C.D and Russel R.L, 1975, Anal. Biochem, 64, 229-238).

Les formulations testées étaient les suivantes :

### Formulation 1 :

9 mg P.S, 8,5 gm huile de soja, 8,5 gm acide oléiue, 1.0 gm Labrafil M1944Cs, 0.02 gm gallate d'isopropyle 0.02 gm PHT, 0,02 gm dimercaptol and 0,02 gm butyl paraben.

### Formulation 2 :

5 mg P.S, 1 ml Ethanol (95%), 1.0 gm Lipoid-E80, 0,02 gm α-Tocopherol, 0,21 gm stéarylamine et 8,0 gm d'huile de soja.

### Formulation 3 :

6 mg P.S, 3 mg Span 80, 0,6 gm Tween 85, 0,36 gm Stéarylamine et huile d'arachide pour 12 gm.

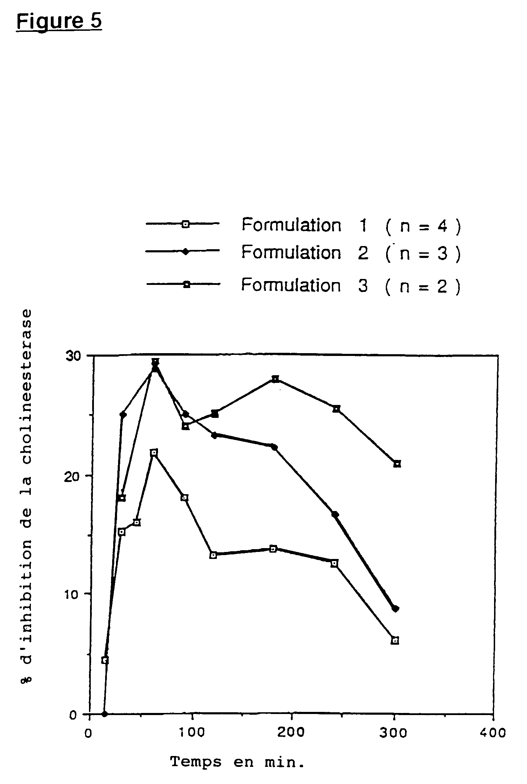

Les résultats de l'inhibition de la choline estérase avec chacune des formulations sont montrés à la figure 5. Ainsi qu'on peut le voir, P.S dissout dans de l'huile de soja sans addition de lipide cationique (formulation 1) était capable d'inhiber environ 20% de l'activité choline estérasique mais l'inhibition n'était pas maintenue pendant de longues périodes de temps. PS dissout dans une formulation d'huile de soja contenant le lipide cationique continuait à maintenir au-dessus de 20% d'inhibition d'activité choline estérasique même trois heures après administration du médicament. L'inhibition la plus prononcée, de plus de 20% pendant plus de 5 heures, était atteinte avec l'utilisation de l'huile d'arachide comme vecteur huileux en combinaison avec le lipide cationique.

### EXEMPLE VIII

### Effet de l'insuline administrée oralement sur le taux de glucose des rats diabétiques.

### Méthodes expérimentales

On a injecté à des rats mâles Sabra pesant 200 g i.p la streptozocine à un dosage de 100 mg/kg, préparé en dissolvant la streptozocine dans du sérum physiologique. Le pH du sérum physiologique était ajusté à 4,4 avec une solution 0.1N d'acide citrique.

La streptozocine affecte le métabolisme du glucose, et à cette dose vide, l'insuline endogène en rendant les rats diabétiques. L'effet diabétogénique a été déterminé 24 heures après injection, en surveillant les taux de glucose dans le sang, en utilisant le test avec un ruban de réactif : une goutte de sang de queue de rat a été appliqué sur le Glucostix^{(TM)} (Miles Ltd, Ames Division, Slough, England) test. Le taux de glucose était détrminé en utilisant le Glucometer II (Miles Lab, Ames Division, Elkhart, USA) selon les instructions du fabricant. Il doit être souligné que la limite maximum du glucomètre est de 400 mg/dl.

Les rats ont été anesthésiés avec de l'éther et la partie abdominale a été rasée. En utilisant un scalpel, on a pratiqué une petite ouverture dans les muscles abdominaux et le peritoine, et l'aire jéjunale des intestins a été sortie et lavée avec du sérum physiologique.

Les diverses formulations lipidiques ont été tout d'abord diluées dans de l'eau bi-distillée (1:5), doucement mélangées et immédiatement injectées dans le jéjunum (1 ml de l'émulsion finale résultante contenant 1 mg d'insuline). Les préparations ont été diluées avant administration, en raison du manque de liquides chez le rat. Les muscles du péritoine et la peau ont ensuite été cousus. Le temps "0" a été choisi avant l'administration du médicament.

Le restant de l'expérience a été effectué alors que les animaux étaient éveillés et du sang a été prélevé de la queue des rats à des intervalles de temps donnés.

Les formulations testées étaient les suivantes :
**Formulation positive** : la formulation SEOF/insuline montrée au tableau VI (N°4), qui fournit des gouttelettes d'émulsion chargées positivement.
**Formulation négative** : la même formulation que la formulation positive sans l'oleylamine, qui fournit des gouttelettes d'émulsion chargées négativement.
**Formulation à blanc** : une formulation similaire à la formulation positive sans insuline.
**Suspension aqueuse d'insuline** : une suspension d'insuline dans le sérum physiologique.
**Préparation d'insuline commerciale** : préparation d'insuline mise sur le marché par NOVO.

Les résultats sont montrés au tableau IX suivant :

**Tableau IX**

| **L'effet des différentes formulations lipidiques sur les taux sanguins de glucose après administration intra-jéjunale** **Taux sanguins de glucose mg/dl (% reduction*********)** | | | | |
|---|---|---|---|---|
| Temps (min) | Formulation positive N = 6 | Emulsion à blanc N = 3 | Suspension aqueuse d'insuline N = 4 | Insuline NOVO 8 unités commercialisée i.p injection N = 1 |
| 0 | > 400 | > 400 | > 400 | > 400 |
| 15 | | | | 253 (37 %) |
| 45 | | | 325 (18 %) | 165 (59 %) |
| 60 | 260 (29 %) | 390 (2.5 %) | | 164 (59 %) |
| 90 | 246 (31 %) | 392 (2 %) | 312 (13 %) | |
| 120 | 237 (30 %) | 374 (6.5 %) | 339 (10 %) | 105 (74 %) |
| 150 | | 375 (6 %) | | |
| 180 | 255 (25 %) | 365 (9 %) | 332 (12 %) | 109 (73 %) |
| 240 | 350 (10 %) | 396 (1 %) | 387 (5 %) | 100 (75 %) |

| | | | | |
|---|---|---|---|---|
| (*) les pourcentages des taux de glucose dans le sang ont été calculés sur la base de 400 mg/dl au temps O, même si les niveaux réels étaient plus élevés. | | | | |

Il peut être noté des résultats montrés au tableau IX, que la dispersion aqueuse de l'insuline n'était pas significativement active, bien qu'une certaine absorbtion se soit produite comme conséquence de la très haute dose administrée. Il peut être noté que la solution d'insuline mise sur le marché, injectée i.p aux rats diabétiques (8 unités/rat) a réduit notablement le taux de glucose dans le sang montrant la validité de l'utilisation de ces rats comme modèle de comparaison.

La formulation lipidique à blanc qui comprend tous les excipients sauf l'insuline, n'a induit aucun effet hypoglycémique, comme prévu. La formulation positive qui avait des gouttelettes d'émulsion chargées positivement, a permis un effet hypoglycémique bien plus efficace que les effets provoqués par la suspension aqueuse d'insuline.

### EXEMPLE IX

### Concentration sérique de progestérone à la suite d'administration de diverses formulations

La progestérone est le composant endogène lipophile qui n'est pas administré par voie orale en raison des effets importants de premier passage. La capacité du SEOF à améliorer sa biodisponibilité par voie orale a été testée dans la présente étude.

Quatre groupes de rats femelles Sabra, agées de 24-28 jours (6 rats dans un groupe) ont reçu 3 formulations, contenant de la progestérone et une formulation a blanc. Chaque animal a été alimenté avec à 0,5 ml de la formulation appropriée qui contenait, soit aucun médicament, soit 4 mg de progestérone par kilo de poids.

Les formulations testées ont été les suivantes :
**Formulation à blanc** : SEOF, décrit dans l'exemple VI (N°5) diué au 1:50, avec une quantité d'eau doublement distillée (DDW) sans le médicament.
**Suspension :** Progestérone suspendue dans DDW
**Formulation négative :** SEOF, décrit dans l'exemple VI (N°5) dilué au 1:50ème avec DDW, sans oleylamine.
**Formulation positive** : SEOF, décrit dans l'exemple VI (N°5) dilué au 1:50ème avec DDW.

Les concentrations de progestérone ont été déterminées par Radio immuno Assay (RIA) en utilisant une trousse avec un tube recouvert ImmChem^{(TM)}, ICN Biomédicals, Inc. Costa Miesa - Californie USA.

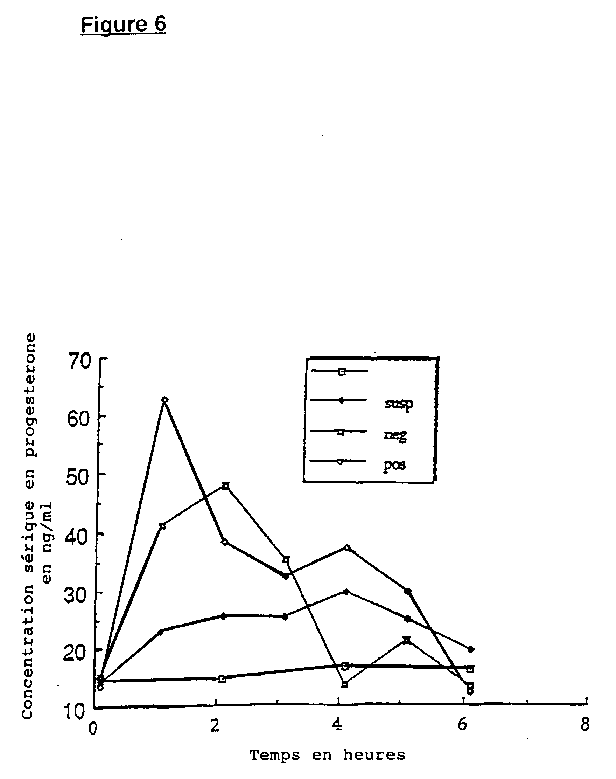

Les résultats sont indiqués à la figure 6. Les paramètres pharmacocinétiques de la progestérone, obtenues à la suite de son administration dans diverses formulations, ainsi que la biodisponibilité relative de ceux du SEOF en comparaison à la suspension sont présentés dans le tableau X.

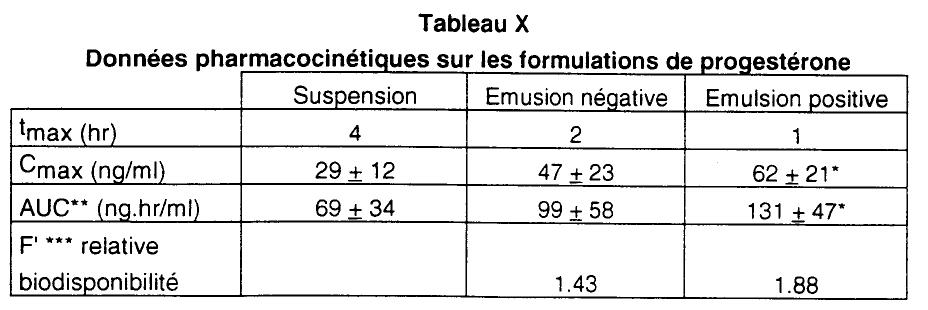

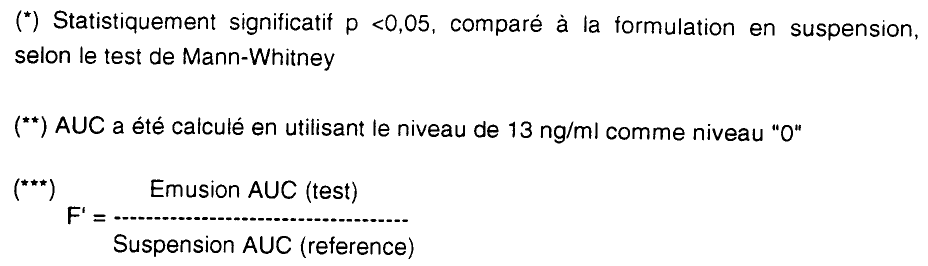

### EXEMPLE X

### Toxicité aiguë du SEOF sur des souris BALB/c

La toxicité aiguë des formulations décrites dans les exemples II - IV, a été examinée sur des souris BALB/c pendant 30 jours. 0.5 ml de chacun des SEOF dilué au 10ème (plus que 300 fois constitue un surdosage) et au 100ème (plus que 30 fois constitue un surdosage) ont été injectés intrapéritonalement au groupe de 4-6 souris. L'état de l'animal a été surveillé pendant plusieurs heures après le processus et ensuite chaque jour, pandant un mois.

Les souris qui ont reçu les eux dilutions de SEOF, décrites dans l'exemple II et la dilution au 100ème de formulation, décrite dans l'exemple IV (contenant 12.5% et 30% de BA) ne montraient aucun signe d'intoxication. La dilution au 10ème de SEOF contenant 12.5% BA n'a causé également aucun effet toxique. D'autre part, on a trouvé que la dilution au 10ème de la formulation, contenant 30% d'alcool benzylique induisait unchoc neurologique immédiat avec amélioration partielle ultérieure et mort des animaux après plusieurs jours. Une dose de moitié de la même formulation a cause encore des symptômes neurotoxiques mais les souris sont restées vivantes.

Comme on le constatera au tableau XI, les mêmes effets toxiques ont été observés également après injection de 1.5% et 3% de solution d'eau BA confirmant son rôle dans la toxicité par surdosage de 300 fois de la formulation.

**Tableau XI**

| **Toxicité aiguë des SEDDS émulsionnés sur des souris BALB/c à la suite d'injection intrapéritonéale** | | | | | |
|---|---|---|---|---|---|
| Formulation (%, w/w) | | Taux de dilution dans l'eau pour injection (vol*) | Nombre d'animaux par groupe | % de survivants après 30 jours | Effets secondaires |
| Tw.80 | 25 | 1:10 (0.5) | 6 | 84 | aucun |
| BA | 12.5 | | | | |
| OA | 3 | 1:100 (0.5) | 6 | 100 | aucun |
| EO | to 100 | | | | |
| Tw.80 | 25 | 1:10 (0.5) | 6 | 0 | choc neurologique |
| BA | 30 | | | | |
| OA | 3 | 1:100 (0.5) | 6 | 100 | aucun |
| EO | to 100 | 1:10 (0.25) | 4 | 100 | choc neurologique |
| BA | 3 | 1:10 (0.5) | 4 | 15 | choc neurologique |
| dans l'eau pour injection | | | | | |
| BA | 1.5 | 1:10 (0.5) | 6 | 100 | choc neurologique |
| dans l'eau pour injection | | | | | |

| | | | | | |
|---|---|---|---|---|---|
| (*) Vol : Volume de l'émulsion résultant injectée intrapéritonal | | | | | |
| (**) Dilution au 10ème : une dose 300 fois plus grande que la dose devant être administrée. | | | | | |

## Revendications

1. Préparation pharmaceutique comprenant une formulation auto-émulsionnable huileuse (SEOF), ledit SEOF comprenant un composant huileux et un agent tensioactif, le SEOF étant caractérisé en ce que le composant huileux contient un vecteur huileux, un lipide cationique et un alcool gras huileux lipophile, l'émulsion huile dans l'eau qui se forme par mélange du SEOF ayant des gouttelettes huileuses chargées positivement et de type submicronique.

2. Préparation pharmaceutique selon la revendication 1 dans laquelle lesdits lipides cationiques du SEOF sont des alcoylamines grasses en C₁₀ à C₂₄ et des alcanoylamines grasses en C₁₂ à C₂₄.

3. Préparation pharmaceutique selon la revendication 2 dans laquelle lesdits lipides cationiques du SEOF sont des alcoylamines grasses en C₁₂ à C₁₈ et des alcanoylamines grasses en C₁₂ à C₁₈.

4. Préparation pharmaceutique selon la revendication 3 dans laquelle ledit lipide cationique du SEOF est la stéarylamine ou l'oleylamine.

5. Préparation pharmaceutique selon l'une des revendications 1 à 4 dans lequel ledit vecteur huileux est formé d'un ou plusieurs composants du groupe qui consiste en une huile de triglycéride dans laquelle la chaîne hydrocarbonée a en moyenne 8 à 12 atomes de carbone (MCT), une huile de triglycéride dans laquelle la chaîne hydrocarbonée a une longueur de chaîne moyenne au-dessus de 12 atomes de carbone (LCT) et un acide gras substitué lipophile.

6. Préparation pharmaceutique selon la revendication 5 dans laquelle ledit LCT est formé d'un ou plusieurs composants du groupe qui consiste en de l'huile d'arachide, de l'huile de carthame, de l'huile de sésame, de l'huile de soja, de l'huile de graine de coton et de l'huile d'olive.

7. Préparation pharmaceutique selon la revendication 5 dans laquelle lesdits dérivés d'acides gras huileux sont des esters d'acides gras avec un alcanol.

8. Préparation pharmaceutique selon la revendication 7 dans laquelle ledit dérivé est de l'oléate d'éthyle.

9. Préparation pharmaceutique selon l'une des revendications 1 à 8 dans laquelle ledit alcool huileux gras du SEOF est un alcool dérivé d'acide gras ou un alcool arylique.

10. Préparation pharmaceutique selon la revendication 9 dans laquelle ledit alcool gras est l'alcool oleylique, l'alcool benzylique ou une combinaison des deux.

11. Préparation pharmaceutique selon la revendication 9 dans laquelle ledit alcool gras est l'alcool éthylique.

12. Préparation pharmaceutique selon l'une des revendications 1 à 11 dans laquelle ledit agent tensioactif du SEOF est un agent tensioactif non-ionique.

13. Préparation pharmaceutique selon la revendication 12 dans laquelle l'agent tensioactif non-ionique est le Tween ^{(R)} ou le Span ^{(R)}.

14. Préparation pharmaceutique selon l'une quelconque des revendications 1 à 13 pour l'administration d'un médicament hydrophobe comprenant un vecteur et une quantité efficace dudit médicament, le vecteur étant un SEOF.

15. Préparation pharmaceutique selon la revendication 14 dans laquelle le médicament est de l'insuline.

16. Préparation pharmaceutique selon la revendication 14 pour l'administration systémique par voie orale dudit médicament.

17. Système de délivrance d'un médicament pour l'administration systémique par voie orale d'un médicament hydrophobe comprenant un SEOF selon l'une des revendications 1 à 13 encapsulé dans une capsule.

18. Système de délivrance d'un médicament selon la revendication 17 dans lequel ladite capsule est une capsule de gélatine molle ou dure.

19. Système de délivrance d'un médicament selon la revendication 17 dans lequel ladite capsule est gastro-protégée.

20. SEOF selon l'une quelconque des revendications 1 à 13 pour l'utilisation dans une préparation pharmaceutique.

21. SEOF selon la revendication 20 pour l'utilisation dans une préparation pharmaceutique pour l'administration systémique par voie orale d'un médicament hydrophobe.

22. Procédé pour la production d'une émulsion qui comprend le fait de mélanger un SEOF selon l'une des revendications 1 à 13 avec un milieu aqueux.

23. Procédé selon la revendication 22 pour la préparation d'une émulsion huile dans l'eau qui contient un médicament hydrophobe, le procédé contenant l'incorporation dudit médicament dans un SEOF selon l'une des revendications 1 à 13.

24. Préparation pharmaceutique de la revendication 14, dans laquelle le médicament est de la Cyclosporine A.

## Claims

1. Pharmaceutical preparation comprising a self-emulsifiable oily formulation (SEOF), the said SEOF comprising an oily component and a surfactant, the SEOF being characterized in that the oily component contains an oily vector, a cationic lipid and a lipophilic oily fatty alcohol, the oil-in-water emulsion which is formed by mixing the SEOF having positively charged oily droplets of submicronic type.

2. Pharmaceutical preparation according to Claim 1, in which the said cationic lipids of the SEOF are C₁₀ to C₂₄ fatty alkylamines and C₁₂ to C₂₄ fatty alkanoylamines.

3. Pharmaceutical preparation according to Claim 2, in which the said cationic lipids of the SEOF are C₁₂ to C₁₈ fatty alkylamines and C₁₂ to C₁₈ fatty alkanoylamines.

4. Pharmaceutical preparation according to Claim 3, in which the said cationic lipid of the SEOF is stearylamine or oleylamine.

5. Pharmaceutical preparation according to one of Claims 1 to 4, in which the said oily vector is formed of one or more components from the group which consists of a triglyceride oil in which the hydrocarbon chain has an average of 8 to 12 carbon atoms (MCT), a triglyceride oil in which the hydrocarbon chain has a mean chain length of more than 12 carbon atoms (LCT) and a lipophilic substituted fatty acid.

6. Pharmaceutical preparation according to Claim 5, in which the said LCT is formed of one or more components from the group which consists of groundnut oil, safflower oil, sesame oil, soybean oil, cottonseed oil and olive oil.

7. Pharmaceutical preparation according to Claim 5, in which the said derivatives of oily fatty acids are esters of fatty acids with an alkanol.

8. Pharmaceutical preparation according to Claim 7, in which the said derivative is ethyl oleate.

9. Pharmaceutical preparation according to one of Claims 1 to 8, in which the said fatty oily alcohol of the SEOF is a fatty-acid-derived alcohol or an aryl alcohol.

10. Pharmaceutical preparation according to Claim 9, in which the said fatty alcohol is oleyl alcohol, benzyl alcohol or a combination of the two.

11. Pharmaceutical preparation according to Claim 9, in which the said fatty alcohol is ethyl alcohol.

12. Pharmaceutical preparation according to one of Claims 1 to 11, in which the said surfactant of the SEOF is a non-ionic surfactant.

13. Pharmaceutical preparation according to Claim 12, in which the non-ionic surfactant is Tween® or Span®.

14. Pharmaceutical preparation according to any one of Claims 1 to 13 for the administration of a hydrophobic medicament comprising a carrier and an effective amount of the said medicament, the carrier being an SEOF.

15. Pharmaceutical preparation according to Claim 14, in which the medicament is insulin.

16. Pharmaceutical preparation according to Claim 14 for the systemic administration by the oral route of the said medicament.

17. Delivery system for a medicament, for the systemic administration by the oral route of a hydrophobic medicament, comprising an SEOF according to one of Claims 1 to 13 encapsulated in a capsule.

18. Delivery system for a medicament according to Claim 17, in which the said capsule is a soft or hard gelatin capsule.

19. Delivery system for a medicament according to Claim 17, in which the said capsule is gastroprotected.

20. SEOF according to any one of Claims 1 to 13, for use in a pharmaceutical preparation.

21. SEOF according to Claim 20 for use in a pharmaceutical preparation for the systemic administration by the oral route of a hydrophobic medicament.

22. Process for the production of an emulsion which comprises mixing an SEOF according to one of Claims 1 to 13 with an aqueous medium.

23. Process according to Claim 22 for the preparation of an oil-in-water emulsion which contains a hydrophobic medicament, the process including the incorporation of the said medicament in an SEOF according to one of Claims 1 to 13.

24. Pharmaceutical preparation of Claim 14, in which the medicament is cyclosporin A.

## Patentansprüche

1. Pharmazeutisches Präparat, umfassend eine selbstemulgierbare ölhaltige Formulierung (SEOF), wobei die SEOF einen ölartigen Bestandteil und ein grenzflächenaktives Mittel enthält, wobei die SEOF dadurch gekennzeichnet ist, daß der ölartige Bestandteil einen ölartigen Träger, ein kationisches Lipid und einen ölartigen lipophilen Fettalkohol enthält, wobei die sich durch Mischen des SEOF bildende Öl-in-Wasser-Emulsion positiv geladene Öltröpfchen vom Submikrontyp aufweist.

2. Pharmazeutisches Präparat nach Anspruch 1, wobei die kationischen Lipide der SEOF aus C₁₀-C₂₄-Fettalkylaminen und C₁₂-C₂₄-Fettalkanoylaminen bestehen.

3. Pharmazeutisches Präparat nach Anspruch 2, wobei die kationischen Lipide der SEOF aus C₁₂-C₁₈-Fettalkylaminen und C₁₂-C₁₈-Fettalkanoylaminen bestehen.

4. Pharmazeutisches Präparat nach Anspruch 3, wobei das kationische Lipid der SEOF Stearylamin oder Oleylamin ist.

5. Pharmazeutisches Präparat nach einem der Ansprüche 1 bis 4, wobei der ölartige Träger gebildet wird aus einem oder mehreren Bestandteilen der Gruppe, die aus einem Triglyceridöl, in welchem die Kohlenwasserstoffkette im Mittel 8 bis 12 Kohlenstoffatome aufweist (MCT), einem Triglyceridöl, in dem die Kohlenwasserstoffkette eine mittlere Kettenlänge über 12 Kohlenstoffatome aufweist (LCT), und einer lipophilen substituierten Fettsäure gebildet wird.

6. Pharmazeutisches Präparat nach Anspruch 5, wobei das LCT aus einem oder mehreren Bestandteilen der Gruppe, die aus Erdnußöl, Safloröl, Sesamöl, Sojaöl, Baumwollsamenöl und Olivenöl besteht, gebildet wird.

7. Pharmazeutisches Präparat nach Anspruch 5, wobei die Derivate ölartiger Fettsäuren Ester von Fettsäuren mit einem Alkanol sind.

8. Pharmazeutisches Präparat nach Anspruch 7, wobei das Derivat Ethyloleat ist.

9. Pharmazeutisches Präparat nach einem der Ansprüche 1 bis 8, wobei der ölartige Fettalkohol der SEOF ein von einer Fettsäure abgeleiteter Alkohol oder ein Arylalkohol ist.

10. Pharmazeutisches Präparat nach Anspruch 9, wobei der Fettalkohol Oleylalkohol, Benzylalkohol oder eine Kombination dieser beiden ist.

11. Pharmazeutisches Präparat nach Anspruch 9, wobei der Fettalkohol Ethylalkohol ist.

12. Pharmazeutisches Präparat nach einem der Ansprüche 1 bis 11, wobei das grenzflächenaktive Mittel der SEOF ein nichtionisches grenzflächenaktives Mittel ist.

13. Pharmazeutisches Präparat nach Anspruch 12, wobei das nichtionische grenzflächenaktive Mittel Tween® oder Span® ist.

14. Pharmazeutisches Präparat nach einem der Ansprüche 1 bis 13 für die Verabreichung eines hydrophoben Arzneimittels, umfassend einen Träger und eine wirksame Menge des Arzneimittels, wobei der Träger eine SEOF ist.

15. Pharmazeutisches Präparat nach Anspruch 14, wobei das Arzneimittel Insulin ist.

16. Pharmazeutisches Präparat nach Anspruch 14 für die systemische Verabreichung des Arzneimittels auf oralem Wege.

17. Arzneimittelabgabesystem für die systemische Verabreichung eines hydrophoben Arzneimittels auf oralem Wege, umfassend eine SEOF gemäß einem der Ansprüche 1 bis 13, eingeschlossen in eine Kapsel.

18. Arzneimittelabgabesystem nach Anspruch 17, wobei die Kapsel eine Weichgelatinekapsel oder Hartgelatinekapsel ist.

19. Arzneimittelabgabesystem nach Anspruch 17, wobei die Kapsel vor dem Magen geschützt ist.

20. SEOF nach einem der Ansprüche 1 bis 13 für die Verwendung in einem pharmazeutischen Präparat.

21. SEOF nach Anspruch 20 für die Verwendung in einem pharmazeutischen Präparat für die systemische Verabreichung eines hydrophoben Arzneimittels auf oralem Wege.

22. Verfahren zur Herstellung einer Emulsion, welches umfaßt, eine SEOF nach einem der Ansprüche 1 bis 13 mit einem wäßrigen Medium zu mischen.

23. Verfahren nach Anspruch 22 für die Herstellung einer Ölin-Wasser-Emulsion, die ein hydrophobes Arzneimittel enthält, wobei das Verfahren das Einbringen des Arzneimittels in eine SEOF nach einem der Ansprüche 1 bis 13 umfaßt.

24. Pharmazeutisches Präparat nach Anspruch 14, wobei das Arzneimittel Cyclosporin A ist.
